(19) **Europäisches Patentamt**

**European Patent Office**

**Office européen des brevets**

(11) Publication number : **0 357 724 B1**

(12) # EUROPEAN PATENT SPECIFICATION

(45) Date of publication of patent specification :
02.12.92 Bulletin 92/49

(51) Int. Cl.⁵ : **A61K 31/725, A61K 31/19**

(21) Application number : **89902474.9**

(22) Date of filing : **10.02.89**

(86) International application number :
**PCT/US89/00544**

(87) International publication number :
**WO 89/07444 24.08.89 Gazette 89/20**

(54) **STABILIZED HEPARIN SOLUTION.**

(30) Priority : **18.02.88 US 157547**

(43) Date of publication of application :
**14.03.90 Bulletin 90/11**

(45) Publication of the grant of the patent :
**02.12.92 Bulletin 92/49**

(84) Designated Contracting States :
**BE CH DE FR GB IT LI NL**

(56) References cited :
**EP-A- 240 464**
**WO-A-85/03202**
**WO-A-88/08004**
**US-A- 4 327 086**

(56) References cited :
**US-A- 4 359 463**
**US-A- 4 734 222**
**CHEMICAL ABSTRACTS, vol. 84, no. 22, 31**
**May 1976, Columbus, OH (US); Y.SAITO et al,**
**no. 155656b**

(73) Proprietor : **BAXTER INTERNATIONAL INC.**
**One Baxter Parkway**
**Deerfield, IL 60015 (US)**

(72) Inventor : **BAES, Michel**
**36, avenue des Constellations**
**B-Brussels (BE)**

(74) Representative : **MacGregor, Gordon et al**
**ERIC POTTER & CLARKSON St. Mary's Court**
**St. Mary's Gate**
**Nottingham, NG1 1LE (GB)**

EP 0 357 724 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

In Rock U.S. Patent No. 4,359,463 a method is described in which initial factor VIII activity normally present in blood collected into a calcium-chelating anticoagulant (for example, CPD or ACD) may be maintained by mixing freshly collected blood or plasma with a calcium-heparin solution in sufficient quantities to restore calcium to substantially normal physiological levels. Typically, the solution used contains calcium chloride and heparin.

Heparin containing solutions for storage in collapsible plastic containers are typically buffered with a phosphate, for example monosodium phosphate. Because of this, difficulties are encountered when calcium ions are added to such heparin solutions in an attempt to provide a stable calcium-heparin solution. Calcium phosphate can easily precipitate from the solution in that circumstance.

The heparin must, however, be buffered in order to be stabilized in solution. Particularly, the heparin must be buffered to withstand a heat sterilization and pasteurization cycle without undue loss of heparin activity. Such is of course needed as a prior step to provide an aseptic heparin solution which may be safely administered to collected blood or plasma.

Other references which discuss the effect of heparin and calcium on factor VIII activity in blood or plasma include articles by Morgenthaler et al. "Influence of Heparin and Calcium Chloride on Assay, Stability and Recovery of Factor VIII" Vox Sang. 48, 8-17, (1985) and the article by Rock et al. entitled "Stability of VIII: C in Plasma: The Dependence on Protease Activity and Calcium". Thrombosis Research 29, 521-535, (1983).

By this invention, a heat-sterilizable, stable, aqueous solution of calcium and heparin may be provided, for use in improving the yields and other process parameters of the collection of factor VIII from blood. The solution typically comprises from 5 to 1000 USP units heparin, preferably sodium heparin, per ml. of solution present, and from 10 to 100 millimoles of calcium lactate per liter of such solution. These solutes are preferably completely dissolved in the solution, sufficient heparin being present to retard clotting when added to blood or plasma.

The calcium lactate present has surprisingly been found to exhibit a double function. First, it is an improved stabilizer for heparin, so that such a solution can be heat sterilized under conventional sterilization cycles and stored with less loss of heparin activity. Specifically, the solution can be stored for a period in excess of six months, or at elevated temperatures a period in excess of three months, without major heparin loss. The solution is nontoxic, and useful for providing improved yields and other improvements in known, conventional processes for isolating factor VIII in a therapeutic dosage form from blood. The preparation of such therapeutic dosage forms is a present day commercial activity, and is a well-known process.

Typically, the solution of this invention contains 15 to 50 millimoles of calcium lactate per liter. Also, the solution typically contains from 50 to 200 USP units of heparin per ml.. The heparin concentration in accordance with this invention may be assayed in accordance with the USP assay for heparin sodium as generally described in the USP official Monographs, Volume 21, pages 481 and 482. This assay method may be modified by measuring the clotting time in an available clot timing apparatus rather than by visually measuring the extent of clotting, for greater accuracy. The data herein was collected by such a modified method.

For use of the solution of this invention, one may add the solution to freshly collected blood or preferably freshly separated blood plasma (separated by any conventional means), for example by means of plasmapheresis using an Autopheresis-C® unit sold by the Fenwal division of Baxter Travenol Laboratories, Inc. One may add from 20 to 50 ml of the solution of this invention per unit of blood or plasma, a unit of plasma being about 600 ml in volume. Typically, the addition step may take place immediately after separation of the plasma into a separate unit, the plasma containing an anticoagulant such as ACD or CPD from the original blood collection process. The addition of the solution of this invention after a substantial amount of plasma has entered the separate plasma container prevents the initial aliquots of plasma from sensing an excessive concentration of solution of this invention, which can sometimes initiate an amount of clotting.

After the plasma has been mixed with the desired amount of solution (for example 40 ml. of solution per unit of plasma) the resulting plasma-solution mixture may be processed by a conventional cryoprecipitation process such as described in U.S. Patent Nos. 3,652,530, or 3,631,018, as described in Rock U.S. Patent No. 4,203,891. The solution of this invention not only exhibits significant advantages in the stabilization of heparin in solution, but it also provides improved yields of clinically useable antihemophilic factor (factor VIII) in many conventional processes for the isolation of antihemophilic factor for therapeutic use.

While lactate ion is used in some peritoneal dialysis solutions and intravenous solutions (for example lactated Ringers solution), and while calcium lactate is known as a stabilizing agent for certain prostaglandin compositions (British Patent No. 1,582,162), calcium lactate has apparently never been used as a stabilizer for heparin in solution. The heparin solutions of the prior art have been less stable in heat resistance and/or shelf life than the solution of this application, so that the advantages of this invention have not previously been readily obtainable.

To illustrate the invention of this application, the following examples are disclosed.

Example 1

To 5 liters of water there is added 2.07 g of heparin sodium (1841 USP units per 30 ml) and 45 g of sodium chloride. Three 1 liter portions of this solution were then separated for further processing. To a first one liter portion (solution 1) there was added 2.94 g of calcium chloride dihydrate plus sufficient sodium hydroxide solution to adjust the pH to 8.5. To a second 1 liter portion (solution 2) there was added 8.97 g of calcium gluconate monohydrate. To the third one liter solution portion (solution 3) was added 6.17 g of calcium lactate pentahydrate. Each of solutions 1-3 thus contained essentially 20 mM per liter of calcium.

From the supply of solutions 1-3, primary blood collection bags (Fenwal division of Baxter Travenol Laboratories, Inc.) were each filled with 30 ml of one of the solutions to provide a large plurality of such bags. Some of the bags were sterilized in a conventional sterilization cycle (115-120°C for 50-75 minutes). The bags were then pouched for storage in aluminum foil bags and pasteurized at 96°C for 170 minutes, prior to assay for heparin in accordance with the assay method referred to above.

The original, bulk, 5 liter heparin solution before sterilization exhibited about 61.4 USP units of heparin per ml.

Referring to solution 1, the nonsterile solution had a pH of 6.40, while the sterilized solution had a pH of 4.44 and assayed for 58.6 USP units of heparin per ml.

With respect to solution 2, the nonsterile solution had a pH of 6.42, while the sterilized solution had a pH of 4.44, and assayed at 51.5 USP units of heparin per ml.

With respect to solution 3, containing, calcium lactate, the nonsterile solution exhibited a pH of 6.85, while the sterilized solution had a pH of 5.61, and assayed for 60.4 USP units of heparin per ml.

Thus it can be seen that the heparin content and pH of solution number 3 was preserved through the sterilization process, when compared with the results of solutions 1 and 2, which failed to prevent significant decrease in the concentration of active heparin present and the pH.

When a unit of separated blood plasma is placed into one of the donor bags containing sterile solution 3, the resulting mixture may be processed with greater facility and yield of isolated factor VIII which is suitable for therapeutic use. Preferably, an amount of solution 3 is added to provide between 0.2 to 40 USP units of heparin per ml. of plasma to be processed.

Example 2

(A) Samples of solution 3 as described in Example 1 above were stored for six months at room temperature in sealed, foil-enclosed blood bags of the type previously described. Initially, the sterilized solution assayed for about 60.4 USP units of heparin per ml. and had a pH of 5.61. At the end of six months, the solutions assayed at about 58.6 USP units of heparin per ml. and had a pH of 5.46. (B) Other containers of solution 3 prepared as in Example 1 above, were stored at 45°C for three months. Initially, the containers assayed at 60.4 USP units of heparin per ml. and a pH of 5.61. After the three months storage, the containers assayed at 62.2 USP units of heparin per ml. (the increase being apparently due to experimental error of the assay procedure) and exhibited a pH of 5.35.

It can be seen that little or no degradation of the heparin or the pH took place during storage, even at elevated temperatures. (C) Another sterilized sample of the solution 3, prepared as in Example 1 above initially had the heparin assay and pH as stated in Example 2 (A). After one month of storage at 45°C, the heparin assay was about 61.9 USP units of heparin per ml., and the solution had a pH of 5.49, showing essentially no degradation of either the heparin content or the pH. To the contrary, a similarly treated, bagged sample of solution 1 showed a heparin assay of 61.6 USP units per ml. After sterilization, the solution exhibited a heparin assay of 60.4 USP units per ml. After one month of storage at 45°C the solution exhibited a heparin assay of about 56.4 USP units per ml. Thus it can be seen that the shelf life of solution 1 is much shorter than the shelf life of solution 3.

Thus, by this invention a stable, aqueous heparin solution is provided, having sufficient calcium lactate to permit heat sterilization of the solution and six month (or more) room temperature storage thereof with no more than about 5 weight percent heparin loss.

The above has been offered for illustrative purposes only, and is not intended to limit the scope of the invention of this application, which is as defined in the claims below.

**Claims**

1.  A stable, aqueous solution which comprises in said solution an effective amount of heparin to retard clotting when added to one unit of blood or plasma, and sufficient calcium lactate to permit heat sterilization of said solution and six month room temperature storage thereof with no more than 5 percent heparin loss.

2. A heat-sterilizable, stable, aqueous solution which comprises in said solution an effective amount of heparin to retard clotting when added to one unit of blood or plasma, and from 10 to 100 millimoles of calcium lactate per liter of said solution.

3. The solution of Claim 1 or 2 in which the pH is 4 to 7.

4. The solution of Claim 1, 2 or 3 which contains 15 to 50 millimoles of calcium lactate per liter.

5. The solution of Claim 1, 2, 3 or 4 in which 5 to 1000 USP units of heparin per ml. are present.

6. The solution of Claim 5 in which from 50 to 200 USP units of heparin per ml. of solution are present.

7. The solution of any preceding claim in which said heparin is the sodium salt thereof.

8. The method of adding to collected blood or blood plasma from 20 to 50 ml., per unit of blood or plasma, of an aseptic, stable, aqueous solution which comprises in said added solution an effective amount of heparin to retard clotting when added to said blood or plasma, and from 10 to 100 millimoles of calcium lactate per liter of said solution, whereby improvements may be obtained in the extraction of factor VIII activity therefrom.

9. The method of Claim 7 in which the solution used is as claimed in any one of Claims 3 to 6.

10. The method of Claim 7 or 8 in which said solution is added to freshly separated blood plasma.

11. The method of adding to collected blood or blood plasma from 20 to 50 ml per unit of blood or plasma of a solution according to Claim 1 in which the added amount of said solution comprises an effective amount of heparin to retard clotting of said blood or plasma.

12. Use in the manufacture of an aqueous solution containing an amount of heparin effective to retard clotting when the solution is added to one unit of blood or plasma, of calcium lactate for the purpose of stabilising the heparin in an amount sufficient to permit heat sterilisation of the solution and six months room temperature storage thereof with no more than 5 percent heparin loss.

**Patentansprüche**

1. Stabile wäßrige Lösung, die in der Lösung folgendes aufweist: eine wirksame Menge Heparin, um ein Gerinnen zu verzögern, wenn sie einer Blut- oder Plasmaeinheit zugesetzt ist, und ausreichend Calciumlactat, um eine Hitzesterilisation der Lösung sowie ein sechsmonatiges Lagern derselben bei Raumtemperatur mit nicht mehr als 5 % Heparinverlust zu gestatten.

2. Hitzesterilisierbare, stabile wäßrige Lösung, die in der Lösung folgendes aufweist: eine wirksame Menge Heparin, um ein Gerinnen zu verzögern, wenn sie einer Blut- oder Plasmaeinheit zugesetzt ist, und 10-100 Millimol Calciumlactat pro Liter der Lösung.

3. Lösung nach Anspruch 1 oder 2, wobei der pH-Wert 4 bis 7 beträgt.

4. Lösung nach Anspruch 1, 2 oder 3, die 15 bis 50 Millimol Calciumlactat pro Liter enthält.

5. Lösung nach Anspruch 1, 2, 3 oder 4, wobei 5 bis 1000 USP-Einheiten Heparin pro ml anwesend sind.

6. Lösung nach Anspruch 5, wobei 50 bis 200 USP-Einheiten Heparin pro ml Lösung anwesend sind.

7. Lösung nach einem der vorhergehenden Ansprüche, wobei das Heparin dessen Natriumsalz ist.

8. Verfahren zum Zusetzen einer aseptischen, stabilen wäßrigen Lösung zu gesammeltem Blut oder Blutplasma von 20 bis 50 ml pro Blut- oder Plasmaeinheit, wobei die zugesetzte Lösung folgendes aufweist: eine wirksame Menge Heparin, um ein Gerinnen zu verzögern, wenn sie dem Blut oder Plasma zugesetzt ist, und 10 bis 100 Millimol Calciumlactat pro Liter der Lösung, wodurch Verbesserungen bei der Extraktion von Faktor-VIII-Aktivität daraus erzielbar sind.

9. Verfahren nach Anspruch 7, wobei die verwendete Lösung die Lösung nach einem der Ansprüche 3 bis 6 ist.

10. Verfahren nach Anspruch 7 oder 8, wobei die Lösung frisch getrenntem Blutplasma zugesetzt wird.

11. Verfahren zum Zusetzen einer Lösung nach Anspruch 1 zu gesammeltem Blut oder Blutplasma von 20 bis 50 ml pro Blut- oder Plasmaeinheit, wobei die zugesetzte Menge der Lösung eine wirksame Menge Heparin aufweist, um ein Gerinnen des Bluts oder Plasmas zu verzögern.

12. Verwendung von Calciumlactat bei der Herstellung einer wäßrigen Lösung, die eine Menge Heparin enthält, die wirksam ist, um ein Gerinnen zu verzögern, wenn die Lösung einer Blut- oder Plasmaeinheit zugesetzt ist, zum Zweck des Stabilisierens des Heparins, in einer Menge, die ausreicht, um eine Hitzesterilisation der Lösung und ein sechsmonatiges Lagern derselben bei Raumtemperatur mit nicht mehr als 5 % Heparin-verlust zu gestatten.

## Revendications

1. Solution aqueuse stable, contenant une quantité efficace d'héparine pour retarder la coagulation quand on l'ajoute à une unité de sang ou de plasma, et suffisamment de lactate de calcium pour permettre la stérilisation à la chaleur de ladite solution et un stockage de celle-ci à la température ambiante pendant six mois, sans perte de plus de 5 % en poids d'héparine.

2. Solution aqueuse, stable et stérilisable à la chaleur, contenant une quantité efficace d'héparine pour retarder la coagulation quand on l'ajoute à une unité de sang ou de plasma, et de 10 à 100 millimoles de lactate de calcium par litre de ladite solution.

3. Solution selon la revendication 1 ou 2, dans laquelle le pH est compris entre 4 et 7.

4. Solution selon la revendication 1, 2 ou 3, contenant de 15 à 50 millimoles de lactate de calcium par litre.

5. Solution selon la revendication 1, 2, 3 ou 4, contenant de 5 à 1 000 unités USP d'héparine par ml.

6. Solution selon la revendication 5, contenant de 50 à 200 unités USP d'héparine par ml de solution.

7. Solution selon une quelconque des revendications précédentes, dans laquelle ladite héparine est le sel de sodium de celle-ci.

8. Procédé d'addition, dans du sang ou du plasma sanguin recueilli, de 20 à 50 ml, par unité de sang ou de plasma, d'une solution aqueuse aseptique et stable qui contient, dans ladite solution additionnée, une quantité efficace d'héparine pour retarder la coagulation quand on l'ajoute audit sang ou audit plasma, et de 10 à 100 millimoles de lactate de calcium par litre de ladite solution, grâce à quoi on peut obtenir des améliorations pour l'extraction de l'activité du facteur VIII de cette solution.

9. Procédé selon la revendication 8, dans lequel la solution utilisée est la solution selon une quelconque des revendications 3 à 6.

10. Procédé selon la revendication 8 ou 9, dans lequel ladite solution est ajoutée à du plasma sanguin fraîchement séparé.

11. Procédé d'addition, dans du sang ou du sérum sanguin recueilli, de 20 à 50 ml, par unité de sang ou de plasma, d'une solution selon la revendication 1, dans lequel la quantité ajoutée de ladite solution contient une quantité efficace d'héparine pour retarder la coagulation dudit sang ou dudit plasma.

12. Utilisation, pour la fabrication d'une solution aqueuse contenant une quantité efficace d'héparine pour retarder la coagulation quand on ajoute la solution à une unité de sang ou de plasma, de lactate de calcium, dans le but de stabiliser l'héparine, en une quantité suffisante pour permettre la stérilisation à la chaleur de la solution et un stockage de celle-ci à la température ambiante pendant six mois, sans perte d'héparine de plus de 5 %.